# EUROPEAN PATENT APPLICATION

(11) **EP 1 834 650 A1**
(43) Date of publication of application: **19.09.2007**
(21) Application number: 05822850.3
(22) Date of filing: 27.12.2005
(51) Int. Cl.: A61K 39/39, A61K 9/16, A61K 31/7088, A61K 35/74, A61K 39/00, A61K 45/00, A61K 47/36, A61P 35/00

(54) **CANCER VACCINE PREPARATION**

(30) Priority: 28.12.2004 JP 2004378447
(71) Applicant: ImmunoFrontier, Inc., Tsu-shi, Mie 514-0061 (JP)
(72) Inventor: SHIKU, Hiroshi, Tsu-shi, Mie 5140061 (JP); KAGEYAMA, Shin-ichi, Tsu-shi, Mie 5140061 (JP)
(74) Representative: Paget, Hugh Charles Edward
(86) International application number: PCT/JP2005/023905
(87) International publication number: WO 2006/077724

(57) **Abstract**

[Problems] Conventional cancer vaccines activate only killer T cells, or killer T cells and helper T cells. Thus, no practical cancer vaccine preparation having an obvious capacity to activate antigen presenting cells simultaneously with them has been accomplished.

[Solution Means] A preparation combining a complex of a cancer antigen and a hydrophobized polysaccharide with an agent, e.g., an extract fromhemolytic streptococcus, which binds to a Toll-like receptor to stimulate the antigen presenting cells activates all of the antigen presenting cells, the killer T cells and the helper T cells which are major immune cells when antigen specific immunity against cancer cells is induced and activated, and exerts a potent vaccinal effect. A vaccine preparation of the present invention induces immune responses to the cancer antigen, in particular, has effects of activation and induction of cytotoxic T cells and helper T cells, and thus, can be used as a therapeutic or preventive vaccine for cancer therapy.

## Description

### [Technical Field]

The present invention relates to a cancer vaccine preparation. More particularly, the present invention relates to a cancer vaccine preparation combining complex fine particles of an antigen protein and a hydrophobized polysaccharide with an agent capable of binding to a Toll-like receptor to stimulate an antigen presenting cell. The cancer vaccine preparation of the present invention activates and induces cytotoxic T cells (hereinafter referred to as "killer T cells") and helper T cells specific to an antigen, and also activates antigen presenting cells (hereinafter referred to as "APC") such as dendritic cells, and therefore is extremely useful as a cancer vaccine.

### [Background Art]

Analyses of the immune response to cancers over the years have revealed the importance of cellular immunity in tumor rejection in hosts. It has been demonstrated that a CD8 positive killer T cell is an effector cell which directly destroys a tumor in many tumor systems and that a CD4 positive helper T cell to regulate the function of the CD8 positive killer T cell is important. Also roles and positioning of the antigen presenting cell mainly including the dendritic cell which presents an antigen to both T cells is stimulated and activated by various adhesion molecules and cytokines have been demonstrated.

The killer T cell which plays the role of directly expelling cancer *in vivo* is activated by reacting its antigen recognition receptor with an antigen peptide of 8 to 10 amino acids bound to an MHC class I molecule present on the surface of the cancer cell. In the late 1980s, antigen peptides recognized by the killer T cells which specifically responded to cancer and genes controlling them were reported for the first time, and subsequently, the antigen peptides recognized by the killer T cells and the genes controlling them have been identified and reported in many human and experimental animal cancer cells.

Meanwhile, the killer T cell requires help by the helper T cell which belongs to another T lymphocyte population. The helper T cell recognizes the peptide of 15 to 20 amino acids bound to an MHC class II molecule as the antigen. The existence of a mechanism has been reported that the helper T cell activates the killer T cell present in the vicinity via an action of cytokines such as IL-2 as well as reacts with the dendritic cell which is an important antigen presenting cell to activate it, and as a result, the dendritic cell effectively stimulates the killer T cell. As such, mechanisms in which the killer T cell, the helper T cell and the dendritic cell are stimulated one another as a result of intercellular interactions and consequently the immune responses to cancer are augmented have been demonstrated.

In order to effectively activate the killer T cell which directly attacks a cancer cell, the role of the helper T cell is important. The activated helper T cell amplifies the activation and proliferation of the killer T cell specific to a cancer antigen through production of cytokines and activation of the dendritic cell. It has also been shown that the helper T cell is critical to contributing to the maintenance of activity of the antigen specific killer T cell. Furthermore, it has also been reported that the presence of the helper T cell is important for the accumulation of killer T cells in a local tumor. Development of polyvalent cancer vaccines comprising not only the antigen recognized by the killer T cell but also the antigen recognized by the helper T cell is anticipated (Non-patent document 1).

The present inventors have developed a cancer vaccine aiming at effectively inducing the activation of the killer T cell together with the activation of the helper T cell. For example, the present inventors made a recombinant HER2 protein which has antigen peptides, namely epitopes, presented to killer T cells as well as those presented to helper T cells, and made CHP-HER2 by complexing the protein with a hydrophobized polysaccharide. It has been demonstrated that this complex is internalized in the dendritic cell via endocytosis, subsequently the recombinant protein or the complex itself migrates in cytoplasm and is presented on the cell surface as an MHC class I molecule-binding antigen peptide through the same process as that for endogenous proteins.

Furthermore, it has been found that the dendritic cell which has incorporated this complex efficiently produces an MHC class II molecule-binding antigen peptide and can specifically activate the helper T cell. Thus, the hydrophobized polysaccharide as the complex with the antigen protein molecule can become an excellent antigen delivery system for inducing T cell immunity (Patent document 1, Non-patent document 2). Actually, the present inventors made the complex of the recombinant HER2 protein with the hydrophobized polysaccharide in a clinical grade, performed a clinical study of a cancer vaccine subjecting patients with HER2 positive refractory cancers, and observed the production of an antigen specific antibody and activation of killer T cells and helper T cells.

APC typified by the dendritic cell plays an important role in action mechanisms of a cancer vaccine as described above, but has been originally thought to play a role in natural immunity as a system which rapidly responds in an early phase of infection of pathogenic microorganisms. However, in recent years, it has been revealed that APC also has a system to recognize a foreign antigen. Multiple Toll-like receptors (hereinafter referred to as "TLR") as functional molecules essential for the recognition by APC have been found, and their functions have attracted attention. That is, when the immature dendritic cell is stimulated by capturing a foreign antigen such as a microorganism, it matures by a signal via TLR and simultaneously augments the expression of the molecules such as CD80, CD83, CD86, MHC class I and MHC class II. These molecules are molecules essential for presenting the antigen to the T cell, or costimulatory molecules and molecules which greatly facilitate the activation and proliferation of the antigen specific T cells. The signal from TLR is believed to be directed at a Th1 type helper cell in differentiation of the helper T cells (Non-patent documents 3 and 4). This suggests that stimulus transduction from the dendritic cell through TLR greatly affects the antigen specific activation and proliferation of killer T cells and helper T cells.

[Patent Document 1] US Patent No. 6,656,481
[Non-Patent Document 1] Hiroshi Shiku, Jikken Igaku 22: 2016-2022, 2004
[Non-Patent Document 2] Ikuta Y. et al., Blood 99: 3717-3724, 2002
[Non-Patent Document 3] Takeuchi O & Akira S., Int. Immunopharmacol. 1: 625-635, 2001
[Non-Patent Document 4] Steinman RM & Pope M. , J. Clin. Invest. 109: 1519-1526, 2002.

### [Disclosure of the Invention]

### [Problems to be Solved by the Invention]

The present inventors performed various trials in the process of research and development of a vaccine CHP-HER2 composed of a cancer antigen protein HER2 and a hydrophobized polysaccharide CHP for the purpose of enhancing vaccinal effects by activating T cells which attack cancer cells. Among them, a granulocyte macrophage colony stimulating factor (hereinafter abbreviated as "GM-CSF") differentiates bone marrow cells into phagocytes such as granulocytes and macrophage and has an action which facilitates chemotaxis, and thus was thought to be effective in the maturation and function expression of antigen presenting cells. Thus, the presence or absence of the action augmenting the vaccinal effect described above was examined. When combined with GM-CSF, HER2 specific killer T cells were detected at a higher frequency compared with the use of CHP-HER2 alone. However, it is not easy to produce or obtain GM-CSF which is extremely expensive, which is problematic.

### [Means for Solving the Problems]

As a result of focusing on and studying agents used for cancer therapy over the years and whose safety such as side effects has been sufficiently found, the present inventors have found for the first time that an extract from hemolytic streptococcus augments the vaccinal effect of cancer antigen-hydrophobized polysaccharide complex fine particles to an extent equivalent to or more than that in GM-CSF.

In recent research reports, the extract from hemolytic streptococcus was reported to act upon multiple TLR and was suggested to potentially activate the dendritic cell. However, it was not clear whether the extract from hemolytic streptococcus augments the effects of the antigen-hydrophobized polysaccharide complex in uptake of antigen by dendritic cells, antigen presentation, and activation of T cells and maintenance of activation. However, according to the present invention, the agent combining the antigen-hydrophobized polysaccharide complex with the extract from hemolytic streptococcus exhibits the vaccinal effect which far surpasses the agent composed of the cancer antigen-hydrophobized polysaccharide complex alone, and can be used as a safe and potent cancer vaccine.
Thus, a cancer vaccine preparation according to the first aspect of the invention combines an agent which is a complex of a cancer antigen protein and the hydrophobized polysaccharide with an agent which binds to a Toll-like receptor to stimulate the antigen presenting cells.

A two-drug kit according to the second aspect of the invention is obtained by independently formulating the agent which is a complex of the cancer antigen protein and the hydrophobized polysaccharide and the agent which binds to the Toll-like receptor to stimulate the antigen presenting cells.
In the above first aspect of the invention or second aspect of the invention, it is preferable that the agent which binds to the Toll-like receptor to stimulate the antigen presenting cells is the extract from hemolytic streptococcus or CpG DNA.
It is also preferable that the polysaccharide which composes the hydrophobized polysaccharide is pullulan or mannan and a hydrophobic group is cholesterol.
It is also preferable that the cancer antigen protein is any of HER2, NY-ESO-1 or MEGA A4.

It is also preferable that the cancer antigen protein is a mixture of two or three of HER2, NY-ESO-1 and MEGA A4.
A method for augmenting the cancer vaccinal effect according to the third aspect of the invention is characterized by substantially simultaneously or continuously administering the agent which is the complex of the cancer antigen protein and the hydrophobized polysaccharide and the agent which binds to the Toll-like receptor to stimulate the antigen presenting cells. In this invention, it is preferable that the agent which binds to the Toll-like receptor to stimulate the antigen presenting cells is the extract from hemolytic streptococcus.

### [Effects of the Invention]

The present invention provides a cancer vaccine preparation combining complex fine particles of the antigen protein and the hydrophobized polysaccharide with the agent which binds to the Toll-like receptor to stimulate the antigen presenting cells. By administering this, it can be expected that the production of an antibody against a cancer antigen and cellular immunity to damage cancer cells are intensified *in vivo* and that this will largely contribute to a medical procedure as one of the effective cancer therapeutic agents which will improve pathology and quality of life in patients with cancer.

### [Best Modes for Carrying Out the Invention]

Subsequently, embodiments of the present invention will be described in detail with reference to the drawing, but the technical scope of the present invention is not limited to the following embodiments, and the present invention can be carried out by various modifications without altering its gist. The technical scope of the present invention extends to any equivalent scope.

The antigen protein, the hydrophobized polysaccharide and the agent which binds to the Toll-like receptor to stimulate the antigen presenting cells used in the present invention include the following.
The antigen protein used in the present invention includes cancer testis antigens such as MAGE, BAGE, GAGE and NY-ESO-1, cancer specific mutant antigens such as CDK-1, MUM-1 and CASP-8, tissue specific antigens such as MART-1, TRP, tyrosinase, gp100, PSA and proteinase 3, high expression tumor antigens such as HER2/neu, CEA and SART1, and viral protein antigens of viruses such as EB virus, papilloma virus and adult leukemia virus.

The polysaccharide in the hydrophobized polysaccharide used in the present invention is not particularly limited as long as it is a macromolecule in which a sugar residue has been bound via a glycoside bond, but preferably pullulan or mannan is used. As the hydrophobic group, those in which 1 to 5 (5% or less by mass ratio) alkyl groups or a sterol group having a single strand or double strands have been introduced per 100 monosaccharides are desirable. When the hydrophobic group is the sterol group, preferably cholesterol can be used. When it is the alkyl group, an alkyl group having 10 to 18 carbon atoms can be used.
The complex of the hydrophobized polysaccharide and the antigen protein can be isolated and purified by mixing aggregated fine particles of the hydrophobized polysaccharide with the antigen protein at room temperature and subsequently treating the mixture by gel chromatography method.

The agent which binds to the Toll-like receptor to stimulate the antigen presenting cells includes killed bacteria such as gram-negative bacteria and gram-positive bacteria or components thereof, e.g., lipopolysaccharide, peptide glycan, nucleic acids, lipid proteins and lipid peptides, and preferably the extract from hemolytic streptococcus (lyophilized powder of streptococcus pyogenes treated with penicillin, brand name: Picibanil) or CpG DNA is used.

The vaccine preparation of the present invention can be made into the preparation having a formulation suitable for parenteral administration such as subcutaneous, intravenous or intramuscular administration by typically mixing the above active components, i.e., the complex of the antigen protein and the hydrophobized polysaccharide and the antigen presenting cell-stimulating agent such as hemolytic streptococcus extract with a pharmaceutically acceptable carrier, or as a kit independently formulating the antigen-hydrophobized polysaccharide complex and the antigen presenting cell-stimulating agent such as hemolytic streptococcus extract. The amount of the vaccine preparation of the present invention to be administered required for immunological sensitization can be appropriately determined. For example, as a standard dosage, approximately 50 µg to 150 µg per dose of the antigen protein is targeted, and the antigen-hydrophobized polysaccharide complex and the hemolytic streptococcus extract as dry microbial cells corresponding thereto is administered as one formulation or as distinct formulations corresponding to 0.01 mg to 1.0 mg. When administered as distinct formulations, it is necessary that the active components of both formulations substantially coexist at an administered site. Therefore, it is desirable to substantially simultaneously or continuously administer both formulations. It is appropriate that the administration is performed 2 to 20 times.

### [Example 1]

### <Augmentation of CHP-HER2 cancer vaccine-induced HER2 antigen specific immune response by hemolytic streptococcus extract (OK432, brand name: Picibanil)>

Female BALB/C mice aged 6 weeks were immunized by the following 4 different methods.
1. CHP-HER2 (20 µg) alone was subcutaneously injected.
2. A mixture of 20 µg CHP-HER2 and 1 µg GM-CSF was subcutaneously injected.
3. A mixture of 20 µg CHP-HER2 and 0.1 KE OK432 (corresponding to 0.01 mg of the dry microbial cells) was subcutaneously injected.
4. A mixture of 20 µg CHP-HER2 and an equivalent amount of incomplete Freund's adjuvant (IFA) was subcutaneously injected.

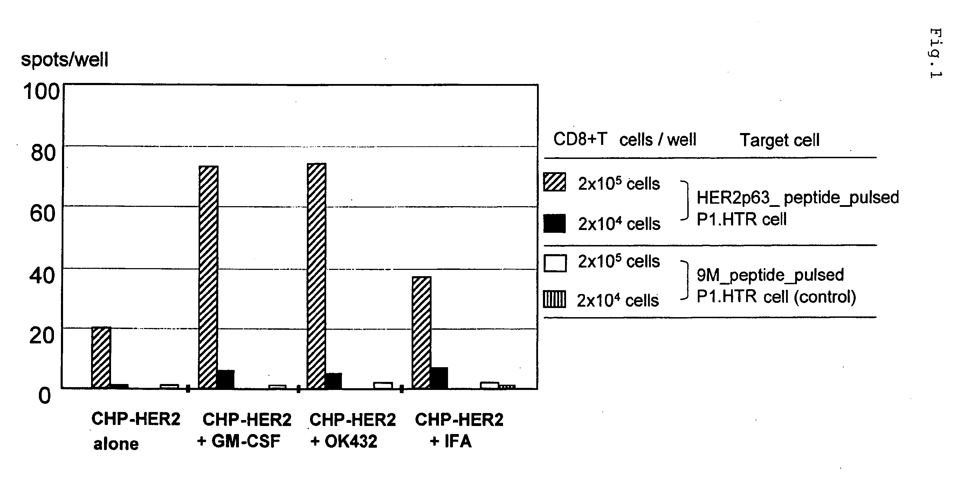

The mouse was immunized by subcutaneously injecting a total volume of 100 µL at a dorsal site twice at one week intervals. One week after the final immunization, spleen cells were collected, CD8 positive cells were isolated, and ELISPOT (enzyme-linked immunospot) assay was performed. As a target cell, P1.HRT cell pulsed with p63-71 peptide which was a CTL epitope derived from HER2 was used. As a control target, P1.HRT cell pulsed with 9m peptide derived from ERK2 was used. As shown in FIG. 1, HER2 specific CD8 positive T cells were detected at 3 to 4 times higher frequency in the GM-CSF combined group and OK432 combined group than in the group administered with CHP-HER2 alone. Meanwhile, in the incomplete Freund's adjuvant combined group, the augmentation effect was about 2 times.

As in the above, according to the present Example, subjecting BALB/C mice, the HER2 antigen specific T cell immune response induced by subcutaneous inoculation of CHP-HER2 was evaluated by the ELISPOT method in which a releasing capacity of y-interferon from the CD8 positive T cells in spleen cells of mice was observed at a single cell level. As a result, an immunopotentiation effect by OK432 *in vivo* was demonstrated. [Example 2]

Among patients where reoccurrence was observed in the urinary bladder during the period after surgical operation of a renal pelvic tumor, CHP-HER2 was administered to the patients where HER2 had been identified to be expressed at a 2+ level by immunohistological staining (HercepTest, Dako, USA) of the tumor cells from a recurrence site. CHP-HER2 was administered by subcutaneously inoculating the amount corresponding to 300 µg of HER2 protein at two week intervals. After the fourth inoculation, 0.02 mg of OK432 (Chugai Pharmaceutical Co., Ltd., Japan) was administered simultaneously with the inoculation of CHP-HER2. Before the initial inoculation, 14 days after the 4th, 6th, 9th and the 11th inoculations, 50 mL of peripheral blood was collected, and the immune response to the HER2 antigen was examined for specific antibody titers, CD4 positive T lymphocytes and CD8 positive T lymphocytes.
Throughout the entire period until after the 11th inoculation, as an adverse event, only a skin reaction of grade I was observed at the injected site. No exacerbation tendency at the tumor site was observed and the disease could be controlled.

The antibody in blood was quantified by ELISA as follows. That is, 10 ng (50 µL as a liquid) of a partial HER2 protein containing amino acid residue from an amino terminus to position 146 (hereinafter "146HER2") per well was adsorbed to an immunoplate (Nunc, Denmark). After washing and blocking, serum from the patient diluted at a ratio of 1:100 to 1:62500 was added to each well and incubated for 10 hours. After washing, a peroxidase-conjugated anti-human IgG (H + LH chain) goat antibody (MBL, Japan) and TMB substrate (Pearce, USA) were added to develop a color, and absorbance OD₄₅₀ at 450 nm was measured using a microplate readermodel 550 (Bio-Rad, USA). As a result, dilution ratios at which the absorbance of more than 0.2 at OD450 was exhibited were 500 times or less before the initial inoculation to the second inoculation, 500 times after the 3rd inoculation, 2,500 times after the 4th inoculation and 12,500 times after the 5th inoculation, indicating that the antibody titer in blood had increased.

HER2 antigen presenting autologous cells subjected to assay of the activated lymphocytes were prepared as follows. That is, the CD4⁺ T lymphocytes were separated from the peripheral blood from the patient using MACS CD4 microbeads (Miltenyi Biotech, USA) and seeded at a density of 1 to 2 x 10⁶ cells per well in RPMI 1640 medium (Gibco, USA) containing 10% human serum, and 10 µg/mL of phytohemagglutinin (Sigma, USA) was added thereto. After 3 days, human IL-2 (Takeda Pharmaceutical Co., Ltd. , Japan) and IL-7 were added to each well and incubated. The cells during 14 to 21 days after the start of the culture were used as the antigen presenting autologous cells T-APC. T-APC at 2.5 to 5 x 10⁶ were mixed with 146HER2 mRNA, and using an electroporation apparatus ECM 830 (BTX, USA), mRNA was introduced into T-APC by an electroporation method to produce HER2 antigen presenting autologous cells (T-APC) transfected with 146HER2 mRNA.

Meanwhile, CD8⁺ T lymphocytes or CD4⁺ T lymphocytes were separated from the peripheral blood from patients using MACS CD8 or MACS CD4 microbeads (Miltenyi Biotech, USA). These T lymphocytes were stimulated by culturing with T-APC transfected with 146HER2 mRNA. From day 8, IL-2 (10 U/mL) was added to the culture medium and culturing was performed.

The HER2 antigen specific T lymphocytes were assayed using the ELISPOT method as follows. A 96-well ELISPOT plate (MAHA S4510 Millipore, USA) was coated with an anti-human interferon γ monoclonal antibody (1-D1K, Mabtech, Sweden), washed and subsequently blocked with RPMI 1640 containing 10% human serum. The sensitized CD8⁺ or CD4⁺ T lymphocytes at 5 x 10⁴ and HER2 antigen presenting T-APC at 1 x 10⁵ were added to each well, and cultured for 22 hours. Then, the plate was washed, and a biotinylated anti-human interferon γ monoclonal antibody (7-B6-1, Mabtech, Sweden) was added. After incubating overnight, streptoavidin-alkali phosphatase complex (Mabtech, Sweden) was reacted for 90 minutes. The plate was washed, stained with an alkali phosphatase complex substrate kit (Bio-Rad), washed and dried. Then, spots were counted using an ELISPOT reader (Carl Zeiss, Germany).

As a result, before the inoculation, after the first inoculation and after the 4th inoculation, no lymphocyte producing antigen reactive interferon γ was observed, but after the 6th inoculation, interferon γ-producing CD8⁺ lymphocytes were significantly observed, and after the 9th and 11th inoculation, both interferon γ-producing CD8⁺ and CD4⁺ T lymphocytes were detected.
As in the above, according to the present Example, as a result of simultaneously inoculating CHP-HER2 and OK432 in patients with cancer and analyzing the immune response specific for HER2 antigen, it was demonstrated that both the production of the HER2 specific antibody and the activation of the HER2 specific T lymphocytes were increased with the increase in the inoculation frequency.

In this manner, according to the present embodiment, by combining the complex fine particles of the antigen protein and the hydrophobized polysaccharide with the agent which binds to the Toll-like receptor to stimulate the antigen presenting cells and inoculating them, the production *in vivo* of the antibody against the cancer antigen is augmented, the cellular immunity which damages the cancer cells is intensified, and thus, this inoculation can greatly contribute to medical procedures as one of the effective cancer therapeutic agents which improve the pathology and quality of life in patients with cancer.

### [Brief Description of the Drawings]

[FIG. 1] is a graph showing an immunopotentiation effect of cancer antigen specific CD8 positive T lymphocytes when CHP-HER2 cancer vaccine was inoculated. In the graph, CHP-HER2 means a hydrophobized polysaccharide-shortened HER2 protein complex, GM-CSF means a granulocyte-macrophage colony stimulating factor, OK432 means Picibanil, and IFA means incomplete Freund's adjuvant.

## Claims

1. A cancer vaccine preparation combining an agent which is a complex of a cancer antigen protein and a hydrophobized polysaccharide with an agent which binds to a Toll-like receptor to stimulate an antigen presenting cell.

2. The cancer vaccine preparation according to claim 1 wherein the agent which binds to the Toll-like receptor to stimulate the antigen presenting cell is an extract from hemolytic streptococcus or CpG DNA.

3. The cancer vaccine preparation according to claim 1 or 2 wherein in the hydrophobized polysaccharide, a component polysaccharide is pullulan or mannan and a hydrophobic group is cholesterol.

4. The cancer vaccine preparation according to any of claims 1 to 3 wherein the cancer antigen protein is any of HER2, NY-ESO-1 or MAGE A4.

5. The cancer vaccine preparation according to any of claims 1 to 4 wherein the cancer antigen protein is a mixture of two or three of HER2, NY-ESO-1 and MAGE A4.

6. A two-drug kit independently formulating an agent which is a complex of a cancer antigen protein and a hydrophobized polysaccharide and an agent which binds to a Toll-like receptor to stimulate an antigen presenting cell.

7. The two-drug kit according to claim 6 wherein the agent which binds to the Toll-like receptor to stimulate the antigen presenting cell is an extract from hemolytic streptococcus or CpG DNA.

8. The two-drug kit according to claim 6 or 7 wherein in the hydrophobized polysaccharide, a component polysaccharide is pullulan or mannan and a hydrophobic group is cholesterol.

9. The two-drug kit according to any of claims 6 to 8 wherein the cancer antigen protein is any of HER2, NY-ESO-1 or MAGE A4.

10. The two-drug kit according to any of claims 6 to 9 wherein the cancer antigen protein is a mixture of two or three of HER2, NY-ESO-1 and MAGE A4.

11. A method for augmenting a cancer vaccinal effect **characterized by** substantially simultaneously or continuously administering an agent which is a complex of a cancer antigen protein and a hydrophobized polysaccharide and an agent which binds to a Toll-like receptor to stimulate an antigen presenting cell.

12. The method for augmenting the cancer vaccinal effect according to claim 11 wherein the agent which binds to the Toll-like receptor to stimulate the antigen presenting cell is an extract from hemolytic streptococcus.
